Europäisches Patentamt

(19) European Patent Office

Office européen des brevets

(11) Veröffentlichungsnummer : **0 171 447**
**B1**

(12) **EUROPÄISCHE PATENTSCHRIFT**

(45) Veröffentlichungstag der Patentschrift :
07.03.90

(51) Int. Cl.$^5$ : **C 12 P 7/58**

(21) Anmeldenummer : 84109673.8

(22) Anmeldetag : 14.08.84

(54) Verfahren zur bakteriellen Herstellung von Gluconsäure.

(43) Veröffentlichungstag der Anmeldung :
19.02.86 Patentblatt 86/08

(45) Bekanntmachung des Hinweises auf die Patenterteilung : 07.03.90 Patentblatt 90/10

(84) Benannte Vertragsstaaten :
AT DE FR GB IT NL

(56) Entgegenhaltungen :
DE--A-- 1 817 907
US--A-- 3 234 105
ACTA BIOTECHNOL., Band 4, Nr. 4, 1984, Seiten 369-
376; W. BABEL: "Methanol-Assimilation durch ein
acidophiles Bacterium der Gattung Acetobacter"
"Microbial Growth on C1 Compounds" 1984, R.L.
Crawford, R.S. Hanson eds., ASM Washington D.C.
USA, S. 141-146
Die Akte enthält technische Angaben, die nach dem
Eingang der Anmeldung eingereicht wurden und die
nicht in dieser Patentschrift enthalten sind.

(73) Patentinhaber : Akademie der Wissenschaften der
DDR
Otto-Nuschke-Strasse 22/23
DDR-1086 Berlin (DD)

(72) Erfinder : Babel, Wolfgang, Dr.
Fritz-Siemon-Strasse 23
DDR-7024 Leipzig (DD)
Erfinder : Miethe, Dietmar, Dr.
Breitscheidstrasse 8
DDR-7114 Zwenkau (DD)
Erfinder : Iske, Uwe, Dr.
Undinenweg 7
DDR-7030 Leipzig (DD)
Erfinder : Sattler, Karl, Dr.
Eitingonstrasse 2
DDR-7010 Leipzig (DD)
Erfinder : Richter, Hans-Peter, Dr.
Nr. 71
DDR-7271 Pohritzsch (DD)
Erfinder : Schmidt, Jörg, Dipl.-Ing.
Strasse der Aktivisten 12
DDR-4413 Sandersdorf (DD)
Erfinder : Düresch, Rolf, Dr.
August-Bebel-Strasse 96
DDR-4410 Wolfen (DD)

(74) Vertreter : Patentanwälte Beetz sen. - Beetz jun.
Timpe - Siegfried - Schmitt-Fumian
Steinsdorfstrasse 10
D-8000 München 22 (DE)

Jouve, 18, rue St-Denis, 75001 Paris, France

**Beschreibung**

Die Erfindung betrifft ein biotechnisches Verfahren zur Herstellung wäßriger Gluconsäurelösungen durch Oxidation von Glucose mit Hilfe von Bakterien.

Die technische Herstellung von Gluconsäure, die gegenwärtig hauptsächlich in der Lebensmittel- und pharmazeutischen Industrie angewendet wird, erfolgt fast ausschließlich auf mikrobiellem Wegen, wobei meist Stämme von Aspergillus niger benutzt werden. Obwohl die Fähigkeit zur Oxidation von Glucose unter Bildung von Gluconsäure (und $H_2O_2$) auch bei Penicillien und Pullularien sowie vielen Bakteriengattungen, darunter Gluconobacter und Acetobacter (Röhr, M., Kubicek, C. P., J. Kominek : Gluconic Acid. In : Biotechnology, Vol. 3, ed. by H. Dellweg, Verlag Chemie Weinheim 1983, pp. 455-465), bekannt ist, werden in technischen Verfahren bisher lediglich Gluconobacter-Stämme eingesetzt.

Im Zusammenhang mit der Herstellung von SCP (single cell protein) auf der Basis von Methanol war bekannt (Babel, W. : Utilization of $C_1$ Compounds by Acidophiles, in : « Microbial Growth on $C_1$ Compounds », ed. by R. L. Cràwford, R. S. Hanson, ASM, Washington D. C., 1984, pp. 141-146, daß es fakultativ methylotrophe Bakterien der Gattung Acetobacter, bezeichnet als Acetobacter MB 58, gibt, die Glucose unvollständig zu Gluconsäure oxidieren können. Eine Gluconsäure-Produktivität dieser Bakterien, die ursprünglich als Art Methylobacter acidophilus, also als obligat methylotroph, eingeordnet worden war, sowie Bedingungen einer eventuellen Gluconsäureherstellung damit wurden nicht angegeben (vgl. Acta Biotechnol. 4 (1984) 4, 369-376).

Die bekannten mikrobiellen Verfahren haben den Nachteil, daß die Produktionskultur unter sterilen Bedingungen — und nur zum Zwecke der Glucoseoxidation — gewonnen wird und daß sowohl in der Wachstumsphase als auch in der ersten Produktbildungsphase (« Gärphase ») — bis die Enzymsynthese abgeschlossen ist — Sterilität gewahrt und der pH-Wert durch Titration mit Alkali- oder Erdalkalihydroxid zwischen 6 und 7 konstantgehalten werden muß. Diese Notwendigkeit der Neutralisation bedingt einen weiteren Nachteil insofern, als durch die damit eintretende Viskositätserhöhung erhebliche Schwierigkeiten für den weiteren technischen Prozeß (erhöhter Energieaufwand, Aufarbeitung usw.) entstehen (DE-B-18 17 907).

Der Erfindung liegt die Aufgabe zugrunde, ein Verfalren zur Herstellung von Gluconsäure unter unsterilen Bedingungen, ohne Neutralisation des Mediums während der Erzeugung des Produzenten und der Produktbildung und ohne Induktionsphase für die Bildung des Glucose oxidierenden Enzyms herzustellen.

Die Aufgabe wird anspruchsgemäß gelöst. Vorteilhafte Weiterbildungen sind Gegenstand der abhängigen Ansprüche.

Das erfindungsgemäße Verfahren zur Herstellung von Gluconsäure beruht auf der Oxidation von Glucose mit Hilfe von Bakterien der Gattung Acetobacter und ist gekennzeichnet durch Verwendung von Bakterien der Art Acetobacter methanolicus, wobei die Glucose ohne Induktion der Glucoseoxidation im pH-Bereich zwischen 1 und 6 ohne Neutralisation und bei Temperaturen zwischen 10 und 35 ˚C diskontinuierlich oder kontinuierlich bei Verweilzeiten zwischen 5 und 60 h zu freier Gluconsäure oxidiert wird. Besonders vorteilhaft ist die Verwendung des in der Stammsammlung des Zentralinstituts für Mikrobiologie und experimentelle Therapie in Jena (DDR) unter der Bezeichnung IMET B 346 hinterlegten Stamms von Acetobacter methanolicus.

Erfindungsgemäß ist ferner von Vorteil, daß die Produktbildung auch mit geringen Zellkonzentrationen bis hinunter zu 0,5 g.l$^{-1}$ durchgeführt werden kann.

Die verwendete Bakterienart der Gattung Acetobacter unterscheidet sich von den bisher beschriebenen in folgenden Merkmalen :

Sehr gutes Wachstum auf Glucose,
sehr gutes Wachstum auf Gluconaten,
sehr gutes Wachstum auf 2,3-Butandiol,
gutes Wachstum auf Methanol,
Wachstum auf Capronsäure und
fehlendes Wachstum auf Lactat.

Morphologie des Typenstamms :

Große, dicke Stäbchen, mehr oder weniger abgerundet, zuweilen zwei oder mehrere kettenförmig zusammenhängend, beweglich, begeißelt (1-3 Geißeln), Gram-negativ ; Größe : 0,9-1,2 μm × 3,6 μm, daneben kürzere Stäbchen mit 0,9 × 1,8 μm und kokkoide Form (0,9 × 0,9 μm).

Koloniemorphologie :

Runde, leicht erhabene, glattrandige, weiß-gelbliche Kolonien von feucht-schmieriger Konsistenz, Größe nach 48 h 0,5-1 mm.

Durch die Synthese eines Glucose oxidierenden Enzyms sind Bakterien der Art Acetobacter methanolicus in der Lage, Glucose als Substrat zu freier Gluconsäure zu oxidieren.

Als Substrate für die Gluconsäuregewinnung können neben reiner Glucose auch glucosehaltige Gemische, insbesondere Glucose/Fructose-Gemische und/oder alle bei der Hydrolyse pflanzlicher Produkte wie Stärke oder Cellulose anfallenden glucosehaltigen End- und Zwischenprodukte, eingesetzt werden. Während der Produktbildung sollte die aktuelle Glucosekonzentration nicht höher als 25 % sein.

Die Gluconsäurebildung kann nach Zuführung der Bakterienbiomasse in einer wäßrigen Substratlösung vorgenommen werden, was die Vorteile einer hohen Reinheit des Produkts und eines geringen Aufwands bei der Aufarbeitung mit sich bringt. Andererseits kann die Produktsynthese auch nach Auszehren des Wachstumsmediums und Zusatz von Substrat im Fermentationsmedium durchgeführt werden. Die Rückhaltung der für den Oxidationsprozeß notwendigen Bakterienbiomasse bzw. Enzymkonzentration kann auch durch Trägerfixierung bzw. Immobilisierung nach bekannten Verfahren erfolgen.

Eine periodische Erneuerung von Biomasse kann vermieden werden, wenn die zur Produktbildung erforderliche Biomasse analog einem zweistufigen Verfahren dem Prozeß kontinuierlich zugeführt wird. In diesem Falle wird jedoch zur Gewinnung einer zellfreien wäßrigen Gluconsäurelösung eine nachgeschaltete Aufarbeitung erforderlich.

Die Biomassekonzentration kann ferner in der Produktionsphase zur Erzielung hoher spezifischer Produktbildungsraten (bis 6 g.h$^{-1}$.g$^{-1}$) und Ausbeuten (bis 97 %) unter 5 g.l$^{-1}$ gehalten werden. Diese ermöglicht — im Falle der Gewinnung von Gluconsäure für technische Zwecke, z. B. zur Verwendung in der Waschmittelindustrie, eine außerordentlich einfache Aufarbeitung, wobei eine vorherige Abtrennung der Biomasse nicht erforderlich ist. Die wäßrige Gluconsäurelösung (bis 310 g.l$^{-1}$) kann durch bekannte Verfahren unmittelbar anschließend aufkonzentriert werden.

Die Vorteile der Verwendung der neuen Bakterienart bestehen darin, daß sie als Produzent für Gluconsäure unsteril, weil im sauren pH-Bereich wachsend, erzeugt werden kann, sowie darin, daß die Produktbildung sofort, d. h. ohne Verzögerung und nicht mit der z. B. bei Aspergillus niger notwendigen Induktionsphase für das Glucose oxidierende Enzym bei dem für das Wachstum optimalen pH-Wert, aber auch oberhalb pH 4 bis pH 6, nach Zugabe von Glucose einsetzt, was im Vergleich zu den herkömmlichen Verfahren höhere Raum/Zeit-Ausbeuten ermöglicht (bis zu 15 g.h$^{-1}$.l$^{-1}$). Daß der pH-Bereich der Glucoseoxidation den pH-Bereich für das Wachstum dieser acidophilen Bakterien bei weitem überschreitet, bedingt weitere Vorteile : Die Produktbildung kann unsteril durchgeführt werden, ferner ist eine Neutralisation nicht erforderlich. Somit kann die bei den bekannten mikrobiologischen Verfahren, die Gluconsäure im wesentlichen als Gluconat produzieren, nachteilige Viskositätserhöhung vermieden werden.

Bei der Verwendng von Glucose/Fructose-Gemischen bzw. von bei der Hydrolyse von pflanzlichen Produkten wie Stärke oder Cellulose anfallenden glucosehaltigen End-, Neben- und Zwischenprodukten als Substrat ist nach dem erfindungsgemäßen Verfahren eine gleichzeitige Gewinnung von Fructose und Gluconsäure durch selektive mikrobielle Oxidation des Glucoseanteils zu Gluconsäure möglich. Nach Abtrennung der gebildeten Gluconsäure kann die Fructose nach bekannten Verfahren gewonnen oder ohne vorherige Abtrennung des Produzenten für nachfolgende Produktsynthesen, beispielsweise zur Gewinnung von Citronensäure, Verwendung finden. Ein mikrobieller Abbau der Fructose ist hierbei auch nach quantitativem Umsatz der Glucose zu Gluconsäure nicht zu beobachten.

Anhand von Ausführungsbeispielen wird die Erfindung im folgenden näher erläutert :

## Beispiel 1

Zu 4 l einer Suspension des beim Zentralinstitut für Mikrobiologie und experimentelle Therapie, Jena, DDR, unter der Hinterlegungsbezeichnung IMET B 346 hinterlegten Bakterienstamms in Wasser Bakterientrockenmasse 50 g/l, pH-Wert 4,2) wurden in einem Rührkesselreaktor von 15 l Gesamtvolumen zu Beginn 800 g Glucosemonohydrat (Ausgangskonzentration an Glucose 15,4 %) und nach 21 und 35 h weitere 500 g Glucosemonohydrat zugegeben.

Folgende Verfahrensparameter wurden eingehalten :

Rührerdrehzahl : 800 min$^{-1}$ ;
Temperatur : 30 °C ;
Belüftungsrate : 10 l/h.

Eine Regelung des pH-Werts erfolgte nicht.

Nach 11 h wurde aufgrund spektrophotometrischer Messung eine Gluconsäurekonzentration von 161 g/l ermittelt.

Zu diesem Zeitpunkt betrugen die spezifische Produktbildungsrate 2,9 g.g$^{-1}$.h$^{-1}$, die Produktivität 14,6 g.l$^{-1}$.h$^{-1}$ und die Ausbeute 92 %. Nach 52 h wurde eine Gluconsäurekonzentration von 310 g/l bestimmt. Die Restglucose im Fermentationsmedium betrug < 0,1 %. Dies entspricht einer mittleren Produktivität von 6,0 g.l$^{-1}$.h$^{-1}$, einer mittleren spezifischen Produktbildungsrate von 1,15 g.g$^{-1}$.h$^{-1}$ und einer Ausbeute von 89,9 %. Der zur Oxidation der Glucose benötigte Sauerstoffbedarf betrug in den ersten 20 h 0,12 g Sauerstoff/g Gluconsäure und stieg in den folgenden 30 h auf 0,16 g Sauerstoff/g Gluconsäure an.

## Beispiel 2

Zu 12 l einer Suspension der in Beispiel 1 genannten Mikroorganismen in Wasser (1 g/l Bakterientrockenmasse, pH-Wert 4,5) wurden in einen Rührkesselreaktor von 30 l Gesamtvolumen 2,4 kg Glucosemonohydrat, nach 21 h 1,2 kh und nach 31 h 0,6 kg Glucosemonohydrat zugegeben. Der Versuch wurde analog Beispiel 1 durchgeführt.

Folgende Werte wurden ermittelt :

Nach 21 h :

| | |
|---|---|
| Gebildete Gluconsäure | 121,5 g/l |
| Ausbeute : | 95,4 % |
| Produktivität | 5,7 g.l$^{-1}$.h$^{-1}$ |
| Spezifische Produktbildungsrate : | 5,7 g.g$^{-1}$.h$^{-1}$ |
| Restglucose : | 4,4 % ; |

nach 44,5 h :

| | |
|---|---|
| gebildete Gluconsäure : | 210 g/l |
| Ausbeute : | 91,1 % |
| Restglucose : | 5 % |
| mittlere Produktivität : | 4,7 g.l$^{-1}$.h$^{-1}$ |
| mittlere spezifische Produktbildungsrate : | 4,7 g.g$^{-1}$.h$^{-1}$. |

## Beispiel 3

In einen 30 l Membranrührkesselreaktor wurden 12 l wäßrige Glucoselösung (100 g/l) gegeben und mit 7 g/l der in Beispiel 1 genannten Biomasse angeimpft.

Analog Beispiel 2 wurden nach 21 h und 31 h 1,2 bzw, 0,6 kg Glucose dem Fermentationsmedium zugesetzt. Die Rührerdrehzahl und die Belüftungsrate während der Produktbildung betrugen 100 min$^{-1}$ bzw. 1,4 l/min. Nach einer Fermentationszeit von 45 h wurde eine Gluconsäurekonzentration von 215 g/l erreicht. Zu diesem Zeitpunkt erfolgte die kontinuierliche Zudosierung einer 25,0-%igen wäßrigen Glucoselösung mit einer Verweilzeit von 20 h. Nach ca. 1,5 Verweilzeitperioden stellte sich ein quasistationärer Gleichgewichtszustand bei einer mittleren Gluconsäurekonzentration von 227 g/l, entsprechend einer Produktivität von 11,3 g Säure/l.h, ein. Das gluconsäurehaltige wäßrige Fermentationsmedium wurde zellfrei über ein geeignetes Membranfilter kontinuierlich aus dem Reaktor abgezogen. Zu diesem Zweck wurde mit Beginn der kontinuierlichen Betriebsweise der Reaktordruck auf 0,5 MPa erhöht. Die Glucoserestkonzentration in der zellfreien wäßrigen Gluconsäurelösung betrug 0,5 %.

Nach einer kontinuierlichen Verfahrensführung von etwa 300 h wurde der Prozeß unterbrochen und mit frischer Biomasse erneut gestartet.

## Beispiel 4

Die zur Produktion von Gluconsäure verwendete Bakterienbiomasse des Bakterienstamms IMET B 346 (vgl. Beisp. 1) wurde zentrifugiert, so daß ein Bakterienkonzentrat mit einem Feststoffgehalt von etwa 80 g/kg anfiel. Mit diesem Bakterienkonzentrat wurden in einem 12-l-Rührkesselfermenter 4 l einer Glucose/Fructose-Mischung mit jeweils 95 g/l Glucose und Fructose angeimpft, so daß sich eine Zellkonzentration von 5 g/l ergab. Anschließend wurde dieses Medium bei 30 °C mit einer Belüftungsrate von 10 l/l.h belüftet und mit einer Rührerdrehzahl von 900 min$^{-1}$ gerührt. Nach einer Fermentationsdauer von 11 h war eine vollständige Oxidation der eingesetzten Glucose zu Gluconsäure erfolgt und eine Säurekonzentration von 90,2 g, entsprechend einer Ausbeute von 95 %, bezogen auf Glucose, bei einer Produktivität von 8,2 g Säure/l.h erreicht. Während dieser Produktbildungsphase wurde die aktuelle Sauerstoffkonzentration größer 10 % des Sättigungswerts gehalten. Die Fructose wurde hierbei von den Mikroorganismen nicht angegriffen.

Nach Abtrennung der Biomasse sowie der Gluconsäure kann die fructosehaltige Mutterlauge je nach Verfahrensziel weiterverarbeitet werden.

## Patentansprüche

1. Verfahren zur bakteriellen Herstellung von Gluconsäure durch Oxidation von Glucose mit Hilfe von Bakterien der Gattung Acetobacter unter Durchführung einer Wachstums- und einer Produktionsphase, gekennzeichnet durch folgende Verfahrensmaßnahmen :

(A) Es werden Bacterien der Art Acetobacter methanolicus verwendet ;

(B) vor der Produktionsphase wird keine Induktion der Glucoseoxidation vorgenommen ;

(C) die Produktionsphase wird ohne Neutralisation im pH-Bereich von 1 bis 6 und bei Temperatu-

EP 0 171 447 B1

ren von 10 bis 35° durchgeführt, und

(D) das Verfahren wird diskontinuierlich oder kontinuierlich bei Verweilzeiten zwischen 5 und 60 h ausgeführt.

2. Verfahren nach Anspruch 1, gekennzeichnet durch Verwendung von Bakterien des beim Zentralinstitut für Mikrobiologie und experimentelle Therapie, Jena, DDR, unter der Hinterlegungsbezeichnung IMET B 346 hinterlegten Stamms von Acetobacter methanolicus.

3. Verfahren nach Anspruch 1, gekennzeichnet durch Anwendung einer Zellkonzentration $\geq 0,5\,g.l^{-1}$.

4. Verfahren nach einem der Ansprüche 1 bis 3, dadurch gekennzeichnet, daß neben Glucose als Substrat auch Glucose/Fructose-Gemische und/oder bei der Hydrolyse pflanzlicher Produkte wie Stärke oder Cellulose anfallende glucosehaltige End- und Zwischenprodukte eingesetzt werden.

5. Verfahren nach einem der Ansprüche 1 bis 4, dadurch gekennzeichnet, daß während der Produktbildung die aktuelle Glucosekonzentration nicht höher als 25 % ist und in Abhängigkeit von der erreichten Produktkonzentration Biomasse nachgeführt wird.

6. Verfahren nach einem der Ansprüche 1 bis 4, dadurch gekennzeichnet, daß zur kontinuierlichen Produktbildung der Produzentenstamm bzw. die entsprechende Biomasse zurückgehalten oder zugeführt wird.

**Claims**

1. Process for the bacterial production of gluconic acid by oxidation of glucose with the aid of bacteria of the genus Acetobacter, by carrying out a growth phase and a production phase, characterized by the following process measures :

(A) bacteria of the species Acetobacter methanolicus are used ;

(B) before the production phase no induction of the glucose oxidation in undertaken ;

(C) the production phase is carried out without neutralization in the pH range from 1 to 6 and at temperatures of 10 to 35°, and

(D) the process is carried out discontinuously or continuously at residence times between 5 and 60 hours.

2. Process according to Claim 1, characterized by the use of bacteria of the strain of Acetobacter methanolicus deposited at the Central Institute for Microbiology and Experimental Therapy, Jena, DDR, under the deposition designation IMET B 346.

3. Process according to Claim 1, characterized by use of a cell concentration $\geq 0.5$ g/l.

4. Process according to one of Claims 1 to 3, characterized in that apart from glucose, glucose/fructose mixtures and/or glucose-containing final and intermediate products arising in the hydrolysis of vegetable products such as starch or cellulose are used as substrate.

5. Process according to one of Claims 1 to 4, characterized in that during product formation the actual glucose concentration is not higher than 25 % and biomass is fed subsequently, as a function of the product concentration reached.

6. Process according to one of Claims 1 to 4, characterized in that for continuous product formation the producer strain or the corresponding biomass is held back or fed.

**Revendications**

1. Procédé de fabrication bactérielle d'acide gluconique par oxydation de glucose à l'aide de bactéries du type acetobacter en exécutant une phase de croissance et une phase de production, caractérisé par les mesures de procédé suivantes :

(A) on utilise des bactéries du type acetobacter methanolicus ;

(B) on entreprend aucune induction de l'oxydation du glucose avant la phase de production ;

(C) la phase de production est exécutée sans neutralisation dans la plage de pH allant de 1 à 6 et à des températures allant de 10 à 35°, et

(D) le procédé est exécuté de manière discontinue ou continue, avec des temps de séjours compris entre 5 et 60 heures.

2. Procédé selon la revendication 1, caractérisé par l'utilisation de bactéries de la souche acetobacter methanolicus déposée au Zentralinstitut für Mikrobiologie und experimentelle Therapie, Jena, DDR, sous la désignation du dépôt IMET B 346.

3. Procédé selon la revendication 1, caractérisé par l'utilisation d'une concentration cellulaire $\geq 0,5\,g.l^{-1}$.

4. Procédé selon l'une des revendications 1 à 3, caractérisé en ce que en plus, le glucose pour substrat, on utilise également des mélanges glucose/fructose et/ou des produits finaux et intermédiaire sensibles à teneur en glucose, dans le cas d'hydrolyse de produits végétaux tels que l'amidon ou la cellulose.

5. Procédé selon l'une des revendications 1 à 4, caractérisé en ce que pendant la formation du

5

produit, la concentration réelle n'est pas supérieure à 25 % et que la biomasse est surveillée en fonction de la concentration en produit atteinte.

6. Procédé selon l'une des revendications 1 à 4, caractérisé en ce que pour la formation continue de produit, la souche productrice ou la biomasse correspondante est enlevée ou fournie.